# EUROPÄISCHE PATENTSCHRIFT

(11) **EP 4 420 619 B1**
(45) Veröffentlichungstag und Bekanntmachung des Hinweises auf die Patenterteilung: **03.12.2025**
(21) Anmeldenummer: 24151334.0
(22) Anmeldetag: 11.01.2024
(51) Int. Cl.: A61B 17/16, A61F 2/18, A61B 17/00

(54) **CHIRURGISCHE SCHNEIDEVORRICHTUNG FÜR DIE OTOLOGIE**
SURGICAL CUTTING DEVICE FOR OTOLOGY
DISPOSITIF DE COUPE CHIRURGICAL POUR OTOLOGIE

(30) Priorität: 24.02.2023 DE 102023104616
(43) Veröffentlichungstag der Anmeldung: 28.08.2024
(73) Patentinhaber: Heinz Kurz GmbH, 72144 Dusslingen (DE)
(72) Erfinder: STIEGELE, Thomas, 72585 Riederich (DE); SANDER, Alexander, 79822 Titisee-Neustadt (DE); MERTENS, Matthias, 24568 Kaltenkirchen (DE); GÄCKLE, Markus, 75378 Bad Liebenzell (DE)
(74) Vertreter: Kohler Schmid Möbus Patentanwälte

(56) Entgegenhaltungen:
- EP-B1- 0 483 567
- US-A- 4 221 222
- US-A1- 2006 111 722

## Beschreibung

Die Erfindung betrifft eine medizinische Schneidevorrichtung der Otologie zur Herstellung dünner Gewebe- oder Knorpelscheiben Knorpelscheibe aus körpereigenem Material eines Patienten für otologische Anwendungen, mit einem länglichen, entlang einer Längsachse ausgerichteten Vorrichtungskörper, der an einem ersten Ende einen stielartigen Handgriff und am entgegengesetzten zweiten Ende einen Schneidkopf mit einer Schneidklinge umfasst, welche einen Klingenkörper mit einer scharfen Schneidkante aufweist, wobei die Schneidklinge innerhalb eines in einer Richtung x' quer zur Richtung der Längsachse durchgängig offenen Schneidfensters angeordnet ist, wobei die Schneidklinge im Schneidfenster derart positioniert ist, dass ihre scharfe Schneidkante parallel zu einer Querachse ausgerichtet ist, welche senkrecht zur Längsachse und schräg zur Richtung x' verläuft, wobei die scharfe Schneidkante der Schneidklinge vom Klingenkörper weg axial in Richtung der Längsachse z zum stielartigen Handgriff hin positioniert ist, wobei die Schneidklinge entweder starr oder um die Querachse beweglich mit dem Vorrichtungskörper verbunden ist, und wobei das Schneidfenster und die Schneidklinge in einem starr mit dem Vorrichtungskörper verbundenen Schneidkörper aufgenommen sind.

Eine derartige Vorrichtung ist bekannt aus US 4 221 222 A (= Referenz [0]).

### Hintergrund der Erfindung

Das Herausschneiden von dünnen körpereigenem Gewebe, insbesondere Knorpelscheiben gleichmäßiger Dicken aus einem größeren, z.B. aus der Ohrmuschel, dem Tragus, dem knorpeligen Anteil der oberen Rippenknochen oder dem Nasen-Septum entnommenen Knorpelstück ist für verschiedene medizinische Zwecke eine immer wieder erforderliche Tätigkeit. Beispielsweise kann es notwendig sein, spezielle Eigenschaften des Grundkörpers näher zu untersuchen, insbesondere unter einem Mikroskop. In der Hals-Nasen-Ohren-Heilkunde werden solche dünnen Knorpelscheiben aber auch bei vielen chirurgischen Eingriffen benötigt, etwa im Mittelohr-Bereich zum Abdecken einer Mittelohr-Prothese, zum Wiederaufbau der Gehörgangs-Hinterwand oder zur plastischen Versorgung eines Trommelfell-Defekts. Auch bei vielen nasen-chirurgischen Eingriffen werden derartige dünne Knorpelscheiben eingesetzt, um funktionelle oder ästhetische Korrekturen an der Nase durchzuführen.

### Vorrichtungen zur Bearbeitung von Gewebe- oder Knorpelscheiben aus körpereigenem Material eines Patienten

In der EP 0 483 567 B1 (= Referenz [2]) ist eine Schneidevorrichtung beschrieben, mit welcher dünne Knorpelscheiben einer -in gewissen Grenzen- vorgebbaren Dicke aus einem größeren Knorpelstück rasch, sicher und in gleichmäßiger Qualität herausgeschnitten werden können. Um unterschiedliche Dicken der erzeugten Knorpelscheiben erzielen zu können, müssen allerdings spezielle Distanzblättchen bekannter Dicke in die Schneidevorrichtung eingelegt werden. Diese Distanzblättchen müssen -ebenso wie die Schneidevorrichtung selbst- streng gereinigt und steril gehalten und vor jeder Operation eigens entsprechend behandelt werden, was einerseits zeitaufwändig und andererseits fehleranfällig ist. Bedenkt man, dass sich in einer durchschnittlichen HNO-Klinik drei bis vier Operationssäle befinden und zu Spitzenzeiten an einem Tag fünfzehn bis zwanzig Patienten operiert werden, so kann es sein, dass an einem Tag bis zu fünfzehnmal ein Knorpelschneider bereitgestellt werden muss. Dies ist für die Sterilgutversorgung eine große logistische Herausforderung. Außerdem ist die Handhabung der Distanzblättchen nicht ganz einfach. So ist es zum Beispiel wegen ihrer geringen Größe nur bedingt möglich, sie ausreichend und gut erkennbar zu beschriften, was aber die Grundvoraussetzung dafür ist, dass während der Operation genau das Distanzblättchen mit der jeweils erforderlichen Größe bereitliegt. Auch das korrekte Einlegen und Fixieren der ziemlich kleinen Distanzblättchen in die Schneidevorrichtung erfordert einige Geschicklichkeit.

Um dünne Knorpelscheiben bestimmter unterschiedlicher Dicken in gleichmäßiger Qualität auch ohne die Verwendung der bekannten Distanzblättchen herstellen zu können, wird in der US 2010/0286693 A1 (= Referenz [3]) vorgeschlagen, dass -wie auch bei der bekannten Schneidevorrichtung nach Referenz [2]- die im ersten Arbeitsabschnitt auf der Oberseite des Vorrichtungskörpers angeordnete erste Vertiefung durch einen auf der Oberseite des Deckels angeordneten ersten Vorsprung verschließbar ist, wobei der erste seitliche Begrenzungssteg einen von einer Stirnseite des ersten Abschnitts her geführten, mit einem vorgegebenen ersten Abstand parallel zur Grundfläche der ersten Vertiefung verlaufenden ersten Führungsschlitz zum Einschieben einer Schneidklinge aufweist. Außerdem soll bei der Vorrichtung nach Referenz [3] mindestens eine zweite Halteeinrichtung vorgesehen sein, die einen zweiten Abschnitt mit einer auf der Oberseite des Vorrichtungskörpers angeordneten zweiten Vertiefung aufweist, welche von einem zweiten Begrenzungssteg ganz oder teilweise umrandet und durch einen auf der Oberseite des Deckels angeordneten zweiten Vorsprung verschließbar ist, wobei der zweite seitliche Begrenzungssteg einen von einer Stirnseite des zweiten Abschnitts her geführten, mit einem vorgegebenen zweiten Abstand parallel zur Grundfläche der zweiten Vertiefung verlaufenden zweiten Führungsschlitz zum Einschieben einer Schneidklinge aufweist.

Da die Abstände zwischen dem jeweiligen Führungsschlitz und der entsprechenden Grundfläche der jeweiligen Vertiefung bei unterschiedlichen Halteeinrichtungen verschieden gewählt werden können, lassen sich mit dieser bekannten Schneidevorrichtung mit den unterschiedlichen Halteeinrichtungen Knorpelscheiben bestimmter unterschiedlicher Dicken herstellen, ohne dass dafür die beim früheren Stand der Technik unerlässlichen Distanzblättchen eingesetzt werden müssen. Weiter gibt eine Handhabung dieser Schneidevorrichtung zwischen Daumen und Zeigefinger dem Operateur mehr Sicherheit beim eigentlichen Schneidevorgang, weil alle Teile aufgrund ihrer Geometrie und ihres Designs sicher und kontrolliert zueinander bewegt werden können. Nachteilig bei dieser bekannten Schneidevorrichtung ist jedoch, dass Teile sich nur mit sehr großem Aufwand herstellen lassen. Gerade spritzgusstechnisch hergestellte Produkte haben die Eigenschaft, dass bei sehr dünnen Wandungen große Probleme entstehen können. Dies hat zur Folge, dass sich die Produkte aufgrund der finanziellen Aufwendungen in der Regel nicht wirtschaftlich realisieren lassen.

Die DE 10 2013 105 857 B4 (=Referenz [4]) verbessert diesen Stand der Technik, indem sie eine modifizierte Schneidevorrichtung vorschlägt, bei deren Benutzung allerdings auch für die handhabende Person eine gewisse Verletzungsgefahr besteht, weil die Schneidklinge, bevor sie in den Führungsschlitz eingesteckt wird, frei zugänglich ist und daher den Benutzer bei einer ungeschickten Bewegung in den Finger schneiden kann. Ein weiterer Nachteil dieser bekannten Vorrichtung (sowie übrigens ebenso des weiter oben diskutierten Standes der Technik) liegt darin, dass das Messer zum Abschneiden der gewünschten Knorpelscheibe mit sägenden beziehungsweise hackenden Bewegungen gehandhabt werden muss, was oftmals zu ungleichmäßigen Schneideergebnissen führt.

Eine Schneidevorrichtung mit rotierendem Schneidmesser ist bekannt aus DE 10 2017 111 634 B3 ≈ EP 3 409 233 B1 ≈ US 10,663,375 B2 (=Referenz [5]).

Eine relativ einfache, frühe Schneidevorrichtung in Form eines Einweg-Knorpelschneiders ist beschrieben in EP 2 249 139 B1 ≈ US 8,563,614 B2 (=Referenz [6]).

Eine gegenüber Referenz [6] verbesserte Ausführungsform eines Einweg-Knorpelschneiders ist dargestellt in DE 10 2013 105 857 B4 ≈ EP 2 810 628 B1 ≈ US 9,532,790 B2 (=Referenz [7]).

Für langlebigere und wiederholte Verwendung zeigt die Anmelderin einen Präzisions-Knorpelschneider auf ihrer Homepage unter https://www.kurzmed.com/de/produkte/otologie/instrumente-und-zubehoer.html dessen Anwendung erklärt wird etwa in https://www.youtube.com/watch?v=NrStxT4JOtU (=Referenz [8]).

Sämtlichen Schneidevorrichtungen gemäß den Referenzen [2] bis [8] ist allerdings gemeinsam, dass sie sich jeweils nur mit der Weiterverarbeitung, insbesondere dem präzischen Zuschnitt bereits vorliegender Gewebe- oder Knorpel-Stücke befassen, nicht jedoch mit der "Gewinnung" derselben aus köpereigenem Material eines Patienten mittels eines chirurgischen Eingriffs.

### Re-Implantationen in der Augenheilkunde

Aus dem -bezüglich der für die vorliegende Erfindung generischen Otologie gedanklich relativ fernliegenden- Gebiet der Ophthalmologie, wo nicht Knorpelgewebe, sondern gänzlich anders beschaffenes, sehr weiches beziehungsweise gallertartiges Cornea- oder Macula-Gewebe entnommen und wiedereingesetzt werden kann, sind etwa aus der DE 698 18 043 T2 (=Referenz [9]) chirurgische Mikrokeratome bekannt. Hierbei werden zur chirurgischen Korrektur von Störungen der Augenlinsenbrechung kleine Teile der Hornhaut verwendet, welche mittels eines speziellen Schneidwerkzeugs entnommen werden können.

Die Firma Gebauer Medizintechnik GmbH in D-75242 Neuhausen beschreibt auf Ihrer Internetseite www.gebauermedical.com (=Referenz [10]) ebenfalls Schneidevorrichtungen zur Entnahme von Cornea-Material mittels einer Hobel-artigen Vorwärts-Bewegung auf Druck vom Handgriff der Vorrichtung in Richtung auf einen Schneidkopf.

### Chirurgische Instrumente zum Abschaben und Sammeln von Knochenpartikeln

In der US 2007/0208348 A1 (= Referenz [11]) ist eine Vorrichtung zum Abschaben von dünnen Knochenteilen -nicht jedoch für Gewebe oder Knorpel- mit einer starr mit dem Vorrichtungskörper verbundenen Schneidklinge beschrieben. Bei dieser Vorrichtung ist die Schneidklinge innerhalb eines in einer Richtung x' quer zur Richtung der Längsachse durchgängig offenen Schneidfensters angeordnet, wobei die Schneidklinge im Schneidfenster derart positioniert ist, dass ihre scharfe Schneidkante parallel zu einer Querachse ausgerichtet ist, welche senkrecht zur Längsachse und schräg zur Richtung x' verläuft. Die scharfe Schneidkante der Schneidklinge ist vom Klingenkörper weg axial in Richtung der Längsachse vom stielartigen Handgriff weg positioniert. Das Abschaben der Knochenteile wird durch eine axiale Hobel-Bewegung vom Handgriff weg in Richtung auf das Schneidfenster bewirkt. Durch die Öffnung im Schneidfenster werden dabei die abgehobelten Knochenpartikel in einen Hohlraum innerhalb der Vorrichtung geschoben, wo sie gesammelt werden können.

### Erfindungsspezifischer Stand der Technik aus der Otologie

Singh SP et al. beschreiben in ihrer Publikation "A Comparative Evaluation of Audiological and Graft Uptake Results of Reinforced Sliced Conchal Cartilage Versus Temporalis Muscle Fascia Graft In Type I Tympoplasty", International Journal of Clinical & Experimental Otolaryngology (IJCEO), 2018; 4(1): 96-100 (=Referenz [12]) ebenfalls die Entnahme und Re-Implantation von Knorpelgewebe in der Ohrheilkunde. Für die Entnahme zeigen und beschreiben sie spezielle Knorpelschneider.

Dietrich Plester et al. "Atlas der Ohrchirurgie", Seite 79, Verlag Kohlhammer (1989) ISBN 3170084887 (= Referenz [1]) beschreibt eine chirurgische Schneidevorrichtung der Otologie zur Herstellung einer dünnen Gewebe- oder Knorpelscheibe aus körpereigenem Material eines Patienten für otologische Anwendungen. Diese Vorrichtung besteht gemäß Referenz [1] aus einem chirurgischen Skalpell mit einigen der eingangs definierten Merkmalskomplexen.

Nachteilig hierbei erscheint die zwangsläufig freihändige Anwendung des Skalpells bei der Gewebe- beziehungsweise Knorpel-Entnahme. Die Qualität des Schneidergebnisses ist dabei immer hochgradig von der Tagesform und dem Geschick des Chirurgen abhängig.

Die bereits eingangs genannte Referenz [0] schließlich beschreibt eine für die vorliegende Erfindung generische chirurgische Schneidevorrichtung mit sämtlichen eingangs definierten Merkmalen.

### Aufgabe der Erfindung

Der vorliegenden Erfindung liegt demgegenüber die Aufgabe zugrunde, eine gattungsgemäße otologische Schneidevorrichtung der eingangs beschriebenen Art mit möglichst einfachen technischen Mitteln unaufwändig und kostengünstig dahingehend zu verbessern, dass die oben geschilderten Vorteile der bekannten Schneidevorrichtungen weitestgehend beibehalten, jedoch die genannten Nachteile vermieden werden. Insbesondere soll mit der Erfindung sichergestellt werden, dass der Schneidevorgang auf einfache Weise und unfallsicher durchgeführt werden kann, wobei besonders gleichmäßige Schneideergebnisse mit am Ende qualitativ hochwertigen Schneide-Produkten, vor allem Korpel- oder Gewebe-Plättchen mit definierter, vorgebbarer Schichtdicke auch ohne weitere Nachbearbeitung erzielbar sein sollen.

### Kurze Beschreibung der Erfindung

Diese relativ komplexe Aufgabe wird, ausgehend von den im Oberbegriff des Anspruchs 1 angegebenen Merkmalen, erfindungsgemäß auf ebenso überraschend einfache wie wirkungsvolle und kostengünstig zu realisierende Weise dadurch gelöst, dass der Schneidkörper einen Abstandshalter aufweist, welcher die Eindringtiefe der scharfen Schneidkante in das körpereigene Material des Patienten und damit die Dicke der herausgeschälten dünnen Gewebe- oder Knorpelscheibe definiert; und dass der Abstandshalter einstellbar gestaltet.

Aufgrund des erfindungsgemäßen Abstandshalters lassen sich besonders genaue und in der Regel auch gleich ohne weitere Bearbeitung für eine Re-Implantation beim Patienten verwendbare Chips einer gewünschten Schichtdicke "ernten".

Dies ermöglicht eine Anwendung der erfindungsgemäß otologischen Vorrichtung auf Zug, im Gegensatz zu den Verfahren in der Ophthalmologie (Referenzen [9], [10]) und der Vorrichtung zum Knochenschaben (Referenz [11]), wo jeweils auf Druck gearbeitet wird. Eine derartige feinfühlige Betriebsweise ist gerade bei der chirurgischen "Bearbeitung" von empfindlichem lebenden Gewebe besonders schonend und führt zu geometrisch deutlich präziseren Arbeitsergebnissen.

Durch diesen Aufbau und die damit vorgegebene Funktionsweise kann sichergestellt werden, dass die scharfe Schneidklinge bei Benutzung stets innerhalb des Führungsschlitzes des Schneidfensters verborgen bleibt, so dass kaum eine Verletzungsgefahr für eine Bedienungsperson besteht.

Die Ausrichtung der scharfen Schneidkante parallel zur Querachse der Vorrichtung ermöglicht gegenüber einem einfachen Skalpell eine gleichmäßige, flächige Zugbewegung der Schneidklinge über dem Ort der Entnahme beim Patienten. Dadurch werden im Gegensatz zu den oben geschilderten Vorrichtungen nach dem Stand der Technik ganz erheblich verbesserte Arbeitsergebnisse im Hinblick auf besonders gleichmäßige, reproduzierbare Schnitte des Gewebe- oder Knorpelstücks und der Gewinnung von Scheiben beziehungsweise Schichten mit genau vorgegebener Dicke ermöglicht. Die erfindungsgemäße Gestaltung der Schneidevorrichtung führt im Endeffekt schließlich zu deutlich besseren, feineren und gleichmäßigeren Schneideergebnissen.

Besondere Vorteile der erfindungsgemäßen Vorrichtung gegenüber dem Stand der Technik sind unter anderem:
Einfachheit der Vorrichtung: In den einfachsten Ausführungsformen umfasst die erfindungsgemäße Schneidevorrichtung lediglich den Handgriff und den Schneidkopf. Beide Teile der Vorrichtung sind mit hoher Fertigungsgenauigkeit, aber dennoch ohne größeren technischen Aufwand problemlos herstellbar.

Präzision: Die Möglichkeit einer Schnitttiefenzustellung erlaubt einen Schnitt durch das Patienten-Gewebe, der sich besonders durch gleichmäßige Schnittdicke und Oberflächenqualität auszeichnet.

Sicherheit: Die Klinge ist für den Anwender nicht unmittelbar zugänglich und macht somit eine Schnittverletzung äußerst unwahrscheinlich.
- Einfaches Abtragen von Knorpelschicht
   ∘ geringer Kraftaufwand
      ▪ Über eine ziehende Bewegung resultiert durch die nach oben gerichtete Kraftkomponente (für den Anwendungsfall) ein leichteres Ziehen als beim Schieben mit der nach unten gerichteten Kraftkomponente
- Definierte Schichtdicke
   ∘ Ein zusätzlicher Arbeitsschritt ist nicht nötig. Bislang wird ein Teil herausgeschnitten und anschließend auf die Schichtdicke nochmals mit einem zusätzlichen Instrument angepasst (siehe Referenzen [2] bis [8]).
- Weniger Abtrag von Knorpelmaterial als beim bisherigen Vorgehen bzw. nur im erforderlichen Maße
   ∘ Schonenderes Vorgehen für den Patienten, schonendere OP und besserer Heilungsprozess
   ∘ Es wird lediglich ein "Chip" sozusagen abgeschält/geerntet ohne dass ein größeres Gewebe- oder Knorpel-Stück herausgeschnitten werden muss.
   ∘ Auch handelt es sich bei diesem Vorgehen um einen zeitlich schnelleren Eingriff, da der abgeschälte ("geerntete") Chip nicht weiter in seiner Dicke bearbeitet werden muss.
- Die erfindungsgemäße Schneidevorrichtung kann als steriles Einwegprodukt ausgeführt werden.
   o Eine aufwändige Aufbereitung entfällt dann.

### Bevorzugte Ausführungsformen der Erfindung

Vorteilhaft ist eine Klasse von Ausführungsformen der erfindungsgemäßen Schneidevorrichtung, bei denen das Schneidfenster und die Schneidklinge in einem einstückig mit dem Vorrichtungskörper verbundenen Schneidkörper aufgenommen sind.

Dies ermöglicht eine präzise Dosierung und Weiterleitung der beim Herausschälen eines Gewebe- oder Knorpel-Stückes angewendeten Kraft. Außerdem erleichtert dieser simple Aufbau auch die Fertigung.

Um die im Einzelfall aktuell erforderliche Schichtdicke besonders genau und fein einstellen zu können, ist bei bevorzugten Weiterbildungen der einstellbare Abstandshalter mit einer oder mehreren Distanzscheiben ausgeführt, die insbesondere eine Dicke zwischen 0,1mm und 1mm, vorzugsweise zwischen 0,3mm und 0,4mm, aufweisen, was den häufigsten Anforderungen normalerweise genügt.

Von besonders großem praktischen Vorteil sind auch Weiterbildungen dieser Ausführungsformen der Erfindung, bei welchen der Schneidkopf im Anschluss an den Schneidkörper in Richtung der Längsachse z zum stielartigen Handgriff hin axial versetzt ein in Richtung einer Hochachse x senkrecht zur Längsachse z sowie senkrecht zur Querachse y durchgängiges Sichtfenster aufweist, wie es per se etwa aus der Druckschrift DE 101 38 235 C1 (= Referenz [13]) bekannt ist.

Dieses Sichtfenster ermöglicht dem Chirurgen während der Gewebe- oder Knorpel-Entnahme eine optische Beurteilung und Kontrolle der Entnahme-Region beim Patienten.

Besonders bevorzugt sind auch Ausführungsformen der erfindungsgemäßen, die sich dadurch auszeichnen, dass am freien, dem Schneidkopf entgegengesetzten Ende des stielartigen Handgriffs eine Knorpelstanzvorrichtung angeordnet ist.

Diese ist beispielsweise zur standardisierten Anfertigung eines Stabilisators für Totalprothesen in die Schneidevorrichtung integriert. Sie erlaubt es, kleine Knorpelscheiben intraoperativ schnell und in einem Arbeitsschritt herzustellen. Derartige Transplantate sind etwa zum Abdecken einer Tympanoplastik-Prothese geeignet.

Ergonomisch besonders günstig ist die Gestaltung von Ausführungsformen der Erfindung, bei welchen die Schneidklinge mit der Seite ihrer scharfen Schneidkante um einen Anstellwinkel zwischen 10° und 40°, vorzugsweise etwa um 25°, gegen die z-y-Ebene gekippt im Schneidkörper angeordnet ist.

Ganz besonders kostengünstig in der Herstellung, aber auch ausreichend reproduzierbar und gleichbleibend in der Qualität sind Ausführungsformen der erfindungsgemäßen chirurgischen Schneidevorrichtung, bei welchen die Schneidklinge aus einer handelsüblichen Rasiermesser-Klinge hergestellt ist.

Eine weitere Klasse von vorteilhaften Ausführungsformen der erfindungsgemäßen Schneidevorrichtung zeichnet sich dadurch aus, dass die Schneidklinge aus Edelstahl, vorzugsweise Chirurgenstahl hergestellt ist.

Damit wird die Schneidevorrichtung auch höchsten Anforderungen an die klinische Hygiene gerade im Bereich der Chirurgie gerecht.

Um die Qualität der erfindungsgemäßen Schneidevorrichtung noch weiter zu erhöhen, weist bei bevorzugten Weiterbildungen dieser Klasse von Ausführungsformen die scharfe Schneidkante der Schneidklinge einen beidseitigen Anschliff auf und ist vorzugsweise speziell gehärtet.

Eine weitere Klasse von -von den Materialkosten her etwas aufwändigeren- Ausführungsformen der Erfindung zeichnet sich dadurch aus, dass der gesamte Vorrichtungskörper der Schneidevorrichtung aus Edelstahl, vorzugsweise Chirurgenstahl, gefertigt ist.

Derartige chirurgischen Schneidevorrichtungen können aber beliebig oft und immer wieder nach den höchsten klinischen Hygienestandards sterilisiert und praktisch über einen beliebig langen Zeitraum wiederverwendet werden.

Bei einer alternativen Klasse von in der Regel etwas kostengünstiger herstellbaren und gewichtsmäßig etwas leichteren Ausführungsformen der Erfindung sind Teile der Schneidevorrichtung, insbesondere der stielartige Handgriff, aus Kunststoff gefertigt.

Die erfindungsgemäße Schneidevorrichtung kann insbesondere auch als preisgünstiges Einweg-Produkt ausgeführt werden, welches nach einmaliger Benutzung entsorgt wird. Damit entfällt eine Reinigung und erneute Sterilisation der Vorrichtung. Dies wird mehr und mehr wichtig, da die Verkeimung von chirurgischen Instrumenten ein immer größer werdendes hygienisches Problem innerhalb des Klinikalltags darstellt. Daher ist die erfindungsgemäße Schneidevorrichtung ganz besonders bevorzugt zumindest in Teilen aus einem sterilisierbaren Kunststoff hergestellt. Damit lässt sich die Schneidevorrichtung ganz erheblich preisgünstiger herstellen als die üblichen Vorrichtungen rein aus Metall. Die Anlieferung zur Operation erfolgt dann in einer Steril-Verpackung und die benutzte Schneidevorrichtung kann einfach entsorgt werden. Ein solches steril verpacktes Einmal-Produkt hat außerdem den Vorteil, dass nicht vor jeder Operation eine aufwändige Reinigung und Sterilisation der Schneidevorrichtung erfolgen muss und es minimiert sich auch das Risiko einer Infektion, dass bei einer Sterilgut-Versorgung nicht ausgeschlossen werden kann. Vorzugsweise wird die entsprechenden Teile der Schneidevorrichtung in einem Spritzguss- oder 3D-Druck-Verfahren hergestellt.

Zur weiteren Material- und Gewichtseinsparung kann bei bevorzugten Ausführungsformen der erfindungsgemäßen chirurgischen Schneidevorrichtung der stielartige Handgriff eine hohle oder konkave Struktur aufweisen.

In der Praxis bewähren sich Ausführungsformen der erfindungsgemäßen chirurgischen Schneidevorrichtung, bei welchen die scharfe Schneidkante der Schneidklinge eine balkenartige oder eine sägezahnartige oder eine sichelartige oder eine ovale oder eine runde, konkave oder konvexe Form aufweist, insbesondere auf einem Kreisabschnitt, vorzugsweise auf einem Viertel- oder Halbkreis.

Nützlich für die Anwendung der vorliegenden Erfindung ist ein Verfahren zum Betrieb einer chirurgischen Schneidevorrichtung der oben beschriebenen erfindungsgemäßen Art zur Herstellung einer dünnen Gewebe- oder Knorpelscheibe, welches sich dadurch auszeichnet, dass die scharfe Schneidkante der Schneidevorrichtung auf eine geeignete Körperstelle des Patienten unter leichtem Andruck aufgesetzt wird, und dass durch Ziehen der Schneidevorrichtung in Richtung des Handgriffs eine gewünschte Gewebe- oder Knorpelscheibe besonders feinfühlig aus dem körpereigenem Material des Patienten herausgeschält wird.

Die Anwendung der erfindungsgemäßen chirurgischen Schneidevorrichtung auf Zug (anstatt auf Druck) ist daher besonders schonend für den Patienten und man erhält im Ergebnis auch viel präzisere Chips.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen.

Die einzelnen Merkmale können je einzeln für sich oder zu mehreren in beliebigen Kombinationen bei Varianten der Erfindung verwirklicht sein. Die gezeigten und beschriebenen Ausführungsformen sind nicht als abschließende Aufzählung zu verstehen, sondern haben vielmehr beispielhaften Charakter für die Schilderung der Erfindung.

### Detaillierte Beschreibung der Erfindung und Zeichnung

In der schematischen Zeichnung sind Ausführungsbeispiele der Erfindung dargestellt, welche in der nachfolgenden Beschreibung näher erläutert werden.

Im Einzelnen zeigen:
- Fig. 1a: eine schematische räumliche Darstellung schräg auf die Unterseite des Vorrichtungskörpers einer Ausführungsform der erfindungsgemäßen Schneidevorrichtung;
- Fig. 1b: wie Fig. 1a, jedoch mit Blick schräg von oben auf die Schneidevorrichtung;
- Fig. 2a: die Ausführungsform von Fig. 1b in einer Ansicht von der Seite in Richtung der Querachse y;
- Fig. 2b: wie Fig. 2a, jedoch in einer Ansicht von oben längs der Hochachse x;
- Fig. 2c: wie Fig. 2b, jedoch in einer Ansicht von unten längs der Hochachse x;
- Fig. 2d: wie Fig. 2a, jedoch in einer Ansicht von hinten in Gegenrichtung zur Längsachse z;
- Fig. 2e: wie Fig. 2d, jedoch in einer Ansicht von vorne in Richtung der Längsachse z;
- Fig. 3a: wie Fig. 2a, jedoch in einer Schnittansicht längs der Schnittlinie A-A mit Blick in Richtung der Querachse y;
- Fig. 3b: Detail B der Ausführungsform von Fig. 3a;
- Fig. 3c: wie Fig. 2b, jedoch mit der Schnittlinie A-A in Richtung der Längsachse z für die Schnittansicht in Fig. 3a;
- Fig. 3d: vergrößertes Detail C der Ausführungsform von Fig. 3a;
- Fig. 4a: eine schematische räumliche Darstellung schräg auf die Unterseite des Vorrichtungskörpers einer Ausführungsform der erfindungsgemäßen Schneidevorrichtung ohne Knorpelstanzvorrichtung in Schäl-Stellung mit angedeutetem körpereigenen Knorpelmaterial eines Patienten;
- Fig. 4b: wie Fig. 3a, jedoch in einer Schnittansicht längs der Schnittlinie D-D mit Blick in Richtung der Querachse y in Schäl-Stellung mit angedeutetem körpereigenen Knorpelmaterial eines Patienten;
- Fig. 4c: vergrößertes Detail E der Ausführungsform von Fig. 4b mit angedeuteter aus dem körpereigenen Knorpelmaterial eines Patienten herausgeschälter Gewebe- oder Knorpelscheibe; und
- Fig. 4d: wie Fig. 3c, jedoch mit der Schnittlinie D-D in Richtung der Längsachse z für die Schnittansicht in Fig. 4b.

Die in den Figuren der Zeichnung schematisch dargestellten Ausführungsformen der erfindungsgemäßen **chirurgischen Schneidevorrichtung 10; 20** dienen zur Herstellung dünner **Gewebe- oder Knorpelscheiben 19'** aus **körpereigenem Material 19** eines Patienten. Die Schneidevorrichtung 10; 20 ist für otologische Anwendungen eingerichtet und umfasst einen länglichen, entlang einer **Längsachse z** ausgerichteten **Vorrichtungskörper 11,** der an einem ersten Ende einen stielartigen **Handgriff 12** und am entgegengesetzten zweiten Ende einen **Schneidkopf 13** mit einer **Schneidklinge 14,** welche einen **Klingenkörper 14'** mit einer **scharfen Schneidkante 14"** aufweist.

Die erfindungsgemäße Schneidevorrichtung 10; 20 zeichnet sich dadurch aus, dass die Schneidklinge 14 innerhalb eines in einer **Richtung x' quer zur Richtung der Längsachse z** durchgängig offenen **Schneidfensters 15** angeordnet ist; dass die Schneidklinge 14 im Schneidfenster 15 derart positioniert ist, dass ihre scharfe Schneidkante 14" parallel zu einer **Querachse y** ausgerichtet ist, welche senkrecht zur Längsachse z und schräg zur Richtung x' verläuft; und dass die Schneidklinge 14 entweder starr oder um die Querachse y beweglich mit dem Vorrichtungskörper 11 verbunden ist.

Das Schneidfenster 15 und die Schneidklinge 14 sind in einem starr, insbesondere einstückig, mit dem Vorrichtungskörper 11 verbundenen **Schneidkörper 13'** aufgenommen. Dieser weist einen **Abstandshalter 16** auf, welcher die Eindringtiefe der scharfen Schneidkante 14" in das körpereigene Material 19 des Patienten und damit die Dicke der herausgeschälten dünnen Gewebe- oder Knorpelscheibe 19' definiert. Der Abstandshalter 16 ist einstellbar gestaltet, bei den in der Zeichnung gezeigten Ausführungsbeispielen mit einer oder mehreren **Distanzscheiben 16'.**

Der Schneidkopf 13 weist im Anschluss an den Schneidkörper 13' in Richtung der Längsachse z zum stielartigen Handgriff 12 hin axial versetzt ein in Richtung einer **Hochachse x** senkrecht zur Längsachse z sowie senkrecht zur Querachse y durchgängiges **Sichtfenster 17** auf.

Am freien, dem Schneidkopf 13 entgegengesetzten Ende des stielartigen Handgriffs 12 kann zusätzlich eine **Knorpelstanzvorrichtung 18** in die Schneidevorrichtung 10 integriert sein. Diese dient beispielsweise zur schnellen Anfertigung eines standardisierten Stabilisators für Totalprothesen aus der mit der erfindungsgemäßen Schneidevorrichtung 10 aus dem körpereigenen Material 19 des Patienten herausgeschälten dünnen Gewebe- oder Knorpelscheibe 19'.

Bei besonders langlebigen Ausführungsformen der erfindungsgemäßen Schneidevorrichtung 10; 20 ist der gesamte Vorrichtungskörper 11 aus Edelstahl, vorzugsweise Chirurgenstahl, gefertigt.

Alternativ können bei anderen Ausführungsformen, die sich auch als preisgünstigere und leichtere Einweg-Produkt eignen, Teile der Schneidevorrichtung 10; 20, insbesondere der stielartige Handgriff 12, aus Kunststoff gefertigt sein.

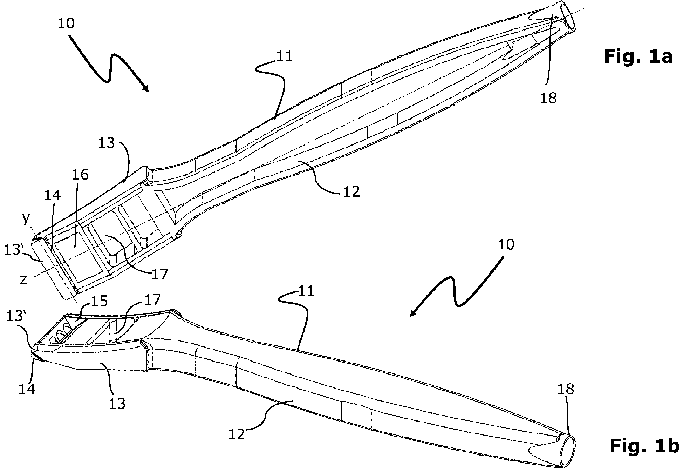

Wie besonders in den Figuren 1a, 3a, 4a und 4b gut zu erkennen ist, kann der stielartige Handgriff 12 aus Gründen der Gewichtseinsparung zudem eine hohle oder konkave Struktur aufweisen.

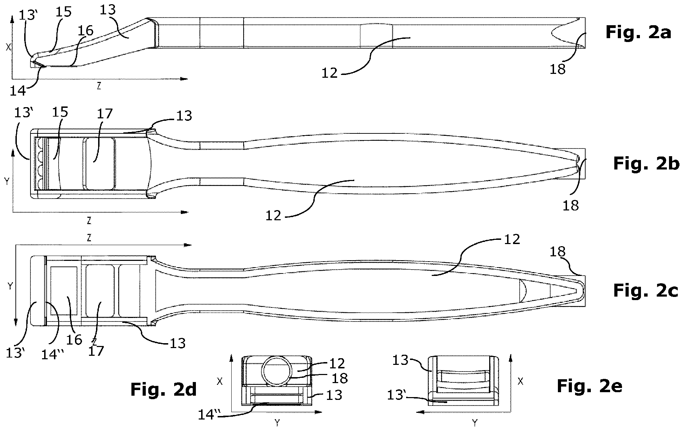

Bei sämtlichen in der vorliegenden Zeichnung gezeigten Ausführungsformen der Schneidevorrichtung 10; 20 weist die die scharfe Schneidkante 14" der Schneidklinge 14 eine balkenartige, parallel zur Querachse y verlaufende Struktur auf, wie insbesondere aus den Figuren 1a und 2c deutlich hervorgeht. Allerdings kann die scharfe Schneidkante 14" bei in der Zeichnung nicht eigens dargestellten Ausführungsformen der Erfindung auch anders geformt sein, etwa eine sägezahnartige oder eine sichelartige oder eine ovale oder eine runde, konkave oder konvexe Form aufweisen, insbesondere auf einem Kreisabschnitt, vorzugsweise auf einem Viertel- oder Halbkreis.

Die Schneidklinge 14 wird in der Regel aus Edelstahl, insbesondere aus Chirurgenstahl, hergestellt sein. Vor allem bei preisgünstigeren Einweg-Produkten kann die Schneidklinge 14 auch aus einer handelsüblichen Rasiermesser-Klinge hergestellt werden. Vorzugsweise ist die scharfe Schneidkante 14" der Schneidklinge 14 speziell gehärtet.

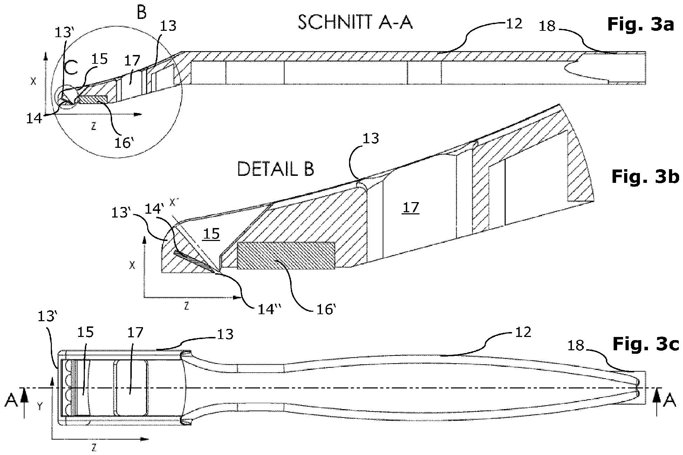

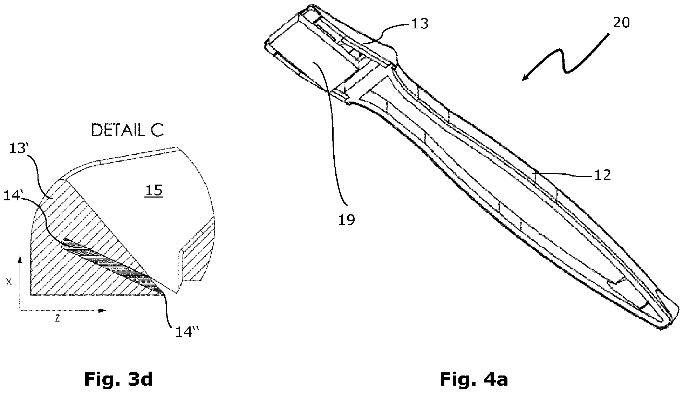

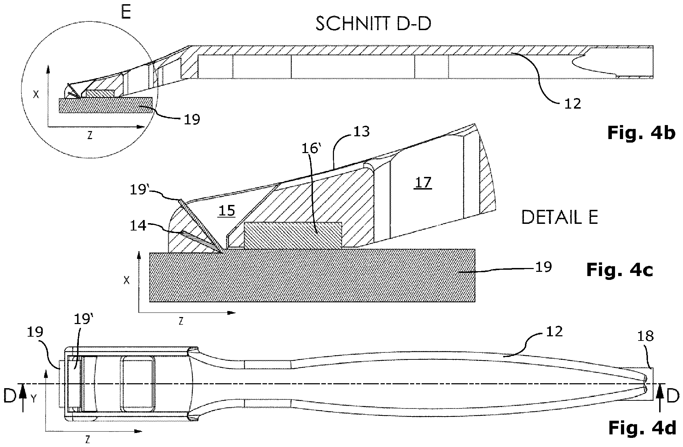

Wie insbesondere in den vergrößerten Detail-Figuren 3b, 3d und 4c gut zu erkennen, kann die scharfe Schneidkante 14" der Schneidklinge 14 einen einseitigen Anschliff aufweisen. Bei in der Zeichnung nicht eigens dargestellten Ausführungsformen der Erfindung kann der Anschliff der Schneidkante aber auch beidseitig ausgeführt sein.

Aus den Figuren 1b, 2a, 3a, 3b, 4b und 4c geht deutlich sichtbar hervor, wie bei besonders bevorzugten Ausführungsformen der erfindungsgemäßen Schneidevorrichtung 10; 20 die Schneidklinge 14 mit der Seite ihrer scharfen Schneidkante 14" um einen Anstellwinkel zwischen 10° und 40°, vorzugsweise etwa um 25°, gegen die z-y-Ebene gekippt im Schneidkörper 13' angeordnet ist. Im Endbereich des Schneidkopfes 13 mit dem Schneidkörper 13' geht der schräge Verlauf zumindest auf der Unterseite dann wieder in einen parallel zur z-y-Ebene verlaufenden Endabschnitt über, welcher beim Abschälen einer dünnen Gewebe- oder Knorpelscheibe 19' auf dem entsprechenden Bereich des zu deren Gewinnung herangezogenen körpereigenen Material 19 des Patienten aufliegt.

Vor allem die Figuren 2a, 3a, 3b, 3d, 4b und 4c verdeutlichen, wie die scharfe Schneidkante 14" der Schneidklinge 14 vom Klingenkörper 14' weg axial in Richtung der Längsachse z zum stielartigen Handgriff 12 hin positioniert ist. Damit lässt sich ein Verfahren zum Betrieb der erfindungsgemäßen chirurgischen Schneidevorrichtung 10; 20 zur Herstellung einer dünnen Gewebe- oder Knorpelscheibe 19' besonders gut durchführen. Dieses ist dadurch gekennzeichnet, dass die scharfe Schneidkante 14" der Schneidevorrichtung 10; 20 auf eine geeignete Körperstelle des Patienten unter leichtem Andruck aufgesetzt wird, und dass durch Ziehen der Schneidevorrichtung 10; 20 in Richtung des Handgriffs 12 eine gewünschte Gewebe- oder Knorpelscheibe 19' aus dem körpereigenem Material 19 des Patienten herausgeschält wird, wie in den Figuren 4a bis 4d angedeutet.

### Bezugszeichenliste:

- 10; 20: chirurgische Schneidevorrichtung
- 11: Vorrichtungskörper
- 12: stielartiger Handgriff
- 13: Schneidkopf
- 13': Schneidkörper
- 14: Schneidklinge
- **14'**: Klingenkörper
- 14";: scharfe Schneidkante
- 15: Schneidfenster
- 16: Abstandshalter
- 16': Distanzscheibe
- 17: Sichtfenster
- 18: Knorpelstanzvorrichtung
- 19: körpereigenes Material des Patienten
- 19': herausgeschälte dünne Gewebe- oder Knorpelscheibe

- x': Richtung quer zur Längsachse
- x: Hochachse
- y: Querachse
- z: Längsachse

### Referenzliste

Für die Beurteilung der Patentfähigkeit in Betracht gezogene Publikationen:
[0] US 4 221 222 A
[1] Dietrich Plester et al. "Atlas der Ohrchirurgie", Seite 79 Verlag Kohlhammer (1989) ISBN 3170084887
[2] EP 0 483 567 B1
[3] US 2010/0286693 A1
[4] DE 10 2013 105 857 B4
[5] DE 10 2017 111 634 B3 ≈ EP 3 409 233 B1 ≈ US 10,663,375 B2
[6] EP 2 249 139 B1 ≈ US 8,563,614 B2
[7] DE 10 2013 105 857 B4 ≈ EP 2 810 628 B1 ≈ US 9,532,790 B2
[8] Internet-Auftritte der Anmelderin mit Präzisions-Knorpelschneider https://www.kurzmed.com/de/produkte/otologie/instrumente-und- zubehoer.html sowie https://www.youtube.com/watch?v=NrStxT4JOtU
[9] DE 698 18 043 T2
[10] Internetseite der Firma Gebauer Medizintechnik GmbH in D-75242 Neuhausen: www.gebauermedical.com
[11] US 2007/0208348 A1
[12] Singh SP et al. "A Comparative Evaluation of Audiological and Graft Uptake Results of Reinforced Sliced Conchal Cartilage Versus Temporalis Muscle Fascia Graft In Type I Tympoplasty", International Journal of Clinical & Experimental Otolaryngology (IJCEO), 2018; 4(1):96-100
[13] DE 101 38 235 C1

## Patentansprüche

1. Chirurgische Schneidevorrichtung (10; 20) der Otologie zur Herstellung einer dünnen Gewebe- oder Knorpelscheibe (19') aus körpereigenem Material (19) eines Patienten für otologische Anwendungen, mit einem länglichen, entlang einer Längsachse (z) ausgerichteten Vorrichtungskörper (11), der an einem ersten Ende einen stielartigen Handgriff (12) und am entgegengesetzten zweiten Ende einen Schneidkopf (13) mit einer Schneidklinge (14) umfasst, welche einen Klingenkörper (14') mit einer scharfen Schneidkante (14") aufweist, wobei die Schneidklinge (14) innerhalb eines in einer Richtung (x') quer zur Richtung der Längsachse (z) durchgängig offenen Schneidfensters (15) angeordnet ist, wobei die Schneidklinge (14) im Schneidfenster (15) derart positioniert ist, dass ihre scharfe Schneidkante (14") parallel zu einer Querachse (y) ausgerichtet ist, welche senkrecht zur Längsachse (z) und schräg zur Richtung (x') verläuft, wobei die scharfe Schneidkante (14") der Schneidklinge (14) vom Klingenkörper (14') weg axial in Richtung der Längsachse (z) zum stielartigen Handgriff (12) hin positioniert ist wobei die Schneidklinge (14) entweder starr oder um die Querachse (y) beweglich mit dem Vorrichtungskörper (11) verbunden ist, und wobei das Schneidfenster (15) und die Schneidklinge (14) in einem starr mit dem Vorrichtungskörper (11) verbundenen Schneidkörper (13') aufgenommen sind,
**dadurch gekennzeichnet,**
**dass** der Schneidkörper (13') einen Abstandshalter (16) aufweist, welcher die Eindringtiefe der scharfen Schneidkante (14") in das körpereigene Material (19) des Patienten und damit die Dicke der herausgeschälten dünnen Gewebe- oder Knorpelscheibe (19') definiert;
und **dass** der Abstandshalter (16) einstellbar gestaltet ist.

2. Schneidevorrichtung nach Anspruch 1, **dadurch gekennzeichnet, dass** das Schneidfenster (15) und die Schneidklinge (14) in einem einstückig mit dem Vorrichtungskörper (11) verbundenen Schneidkörper (13') aufgenommen sind.

3. Schneidevorrichtung nach Anspruch 2, **dadurch gekennzeichnet, dass** der Abstandshalter (16) eine oder mehrere Distanzscheiben (16') umfasst, die insbesondere eine Dicke zwischen 0,1mm und 1mm, vorzugsweise zwischen 0,3mm und 0,4mm, aufweisen.

4. Schneidevorrichtung nach einem der Ansprüche 2 oder 3, **dadurch gekennzeichnet, dass** der Schneidkopf (13) im Anschluss an den Schneidkörper (13') in Richtung der Längsachse (z) zum stielartigen Handgriff (12) hin axial versetzt ein in Richtung einer Hochachse (x) senkrecht zur Längsachse (z) sowie senkrecht zur Querachse (y) durchgängiges Sichtfenster (17) aufweist.

5. Schneidevorrichtung nach einem der vorhergehenden Ansprüche, **dadurch gekennzeichnet, dass** am freien, dem Schneidkopf (13) entgegengesetzten Ende des stielartigen Handgriffs (12) eine Knorpelstanzvorrichtung (18) angeordnet ist.

6. Schneidevorrichtung nach einem der vorhergehenden Ansprüche, **dadurch gekennzeichnet, dass** die Schneidklinge (14) mit der Seite ihrer scharfen Schneidkante (14") um einen Anstellwinkel zwischen 10° und 40°, vorzugsweise etwa um 25°, gegen die z-y-Ebene gekippt im Schneidkörper (13') angeordnet ist.

7. Schneidevorrichtung nach einem der vorhergehenden Ansprüche, **dadurch gekennzeichnet, dass** die Schneidklinge (14) aus einer Rasiermesser-Klinge hergestellt ist.

8. Schneidevorrichtung einem der vorhergehenden Ansprüche,
**dadurch gekennzeichnet, dass** die Schneidklinge (14) aus Edelstahl, vorzugsweise Chirurgenstahl hergestellt ist.

9. Schneidevorrichtung nach Anspruch 7 oder 8, **dadurch gekennzeichnet, dass** die scharfe Schneidkante (14") der Schneidklinge (14) einen beidseitigen Anschliff aufweist und vorzugsweise gehärtet ist.

10. Schneidevorrichtung nach einem der Ansprüche 1 bis 9, **dadurch gekennzeichnet, dass** der gesamte Vorrichtungskörper (11) der Schneidevorrichtung (10; 20) aus Edelstahl, vorzugsweise Chirurgenstahl, gefertigt ist.

11. Schneidevorrichtung nach einem der Ansprüche 1 bis 9, **dadurch gekennzeichnet, dass** Teile der Schneidevorrichtung (10; 20), insbesondere der stielartige Handgriff (12), aus Kunststoff gefertigt sind.

12. Schneidevorrichtung nach einem der vorhergehenden Ansprüche, **dadurch gekennzeichnet, dass** der stielartige Handgriff (12) eine hohle oder konkave Struktur aufweist.

13. Schneidevorrichtung nach einem der vorhergehenden Ansprüche, **dadurch gekennzeichnet, dass** die scharfe Schneidkante (14") der Schneidklinge (14) eine balkenartige oder eine sägezahnartige oder eine sichelartige oder eine ovale oder eine runde, konkave oder konvexe Form aufweist, insbesondere auf einem Kreisabschnitt, vorzugsweise auf einem Viertel- oder Halbkreis.

## Claims

1. A surgical cutting device (10; 20) in otology for the production of a thin fabric or cartilage slice (19') from a patient's own body material (19) for otological applications, having an elongate device body (11) which is aligned along a longitudinal axis (z), and which comprises at a first end a handle-like grip (12) and at the opposite second end a cutting head (13) with a cutting blade (14) which has a blade body (14') with a sharp cutting edge (14"), wherein the cutting blade (14) is arranged within a cutting window (15) which is continuously open in a direction (x') transverse to the direction of the longitudinal axis (z), wherein the cutting blade (14) is positioned in the cutting window (15) in such a manner that its sharp cutting edge (14") is aligned parallel to a transverse axis (y), which extends perpendicular to the longitudinal axis (z) and obliquely to the direction (x'), wherein the sharp cutting edge (14") of the cutting blade (14) is positioned axially away from the blade body (14') in the direction of the longitudinal axis (z) towards the handle-like grip (12), wherein the cutting blade (14) is connected to the device body (11) either rigidly or movably about the transverse axis (y), and wherein the cutting window (15) and the cutting blade (14) are accommodated in a cutting body (13') which is rigidly connected to the device body (11),
**characterized in**
**that** the cutting body (13') has a spacer (16) which defines the penetration depth of the sharp cutting edge (14") into the patient's own body material (19) and thus the thickness of the thin tissue or cartilage slice (19') which is peeled out;
and **that** the spacer (16) is designed to be adjustable

2. The cutting device according to claim 1, **characterized in that** the cutting window (15) and the cutting blade (14) are accommodated in a cutting body (13') which is integrally, connected to the device body (11).

3. The cutting device according to claim 2, **characterized in that** the spacer (16) comprises one or more spacer discs (16'), which particularly have a thickness between 0.1 mm and 1 mm, preferably between 0.3 mm and 0.4 mm.

4. The cutting device according to anyone of claims 2 or 3, **characterized in that** the cutting head (13) has a viewing window (17) following the cutting body (13'), which viewing window is axially offset in the direction of the longitudinal axis (z) towards the handle-like grip (12) and which is continuous in the direction of a vertical axis (x) perpendicular to the longitudinal axis (z) and perpendicular to the transverse axis (y).

5. The cutting device according to anyone of the preceding claims, **characterized in that** a cartilage punching device (18) is arranged at the free end of the handle-like grip (12) opposite the cutting head (13).

6. The cutting device according to anyone of the preceding claims, **characterized in that** the cutting blade (14) is arranged in the cutting body (13') with the side of its sharp cutting edge (14") tilted by a setting angle of between 10° and 40°, preferably approximately 25°, relative to the z-y plane.

7. The cutting device according to anyone of the preceding claims, **characterized in that** the cutting blade (14) is produced from a razor blade.

8. The cutting device according to anyone of the preceding claims, **characterized in that** the cutting blade (14) is produced from stainless steel, preferably surgical steel.

9. The cutting device according to claim 7 or 8, **characterized in that** the sharp cutting edge (14") of the cutting blade (14) has a bevelled polished section on both sides and is preferably hardened.

10. The cutting device according to anyone of claims 1 to 9, **characterized in that** the entire device body (11) of the cutting device (10; 20) is made of stainless steel, preferably surgical steel.

11. The cutting device according to anyone of claims 1 to 9, **characterized in that** parts of the cutting device (10; 20), particularly the handle-like grip (12), are made of plastic.

12. The cutting device according to anyone of the preceding claims, **characterized in that** the handle-like grip (12) has a hollow or concave structure.

13. The cutting device according to anyone of the preceding claims, **characterized in that** the sharp cutting edge (14") of the cutting blade (14) has a beam-like or a sawtooth-like or a sickle-like or an oval or a round, concave or convex shape, in particular on a circular portion, preferably on a quarter or semicircle.

## Revendications

1. Dispositif chirurgical de coupe (10; 20) utilisé en otologie pour produire une fine lamelle de tissu ou de cartilage (19') à partir de matériel autologue (19) d'un patient pour des applications otologiques, comprenant un corps de dispositif allongé (11) aligné le long d'un axe longitudinal (z), qui comprend à une première extrémité une poignée en forme de manche (12) et à la deuxième extrémité opposée une tête de coupe (13) avec une lame de coupe (14) qui présente un corps de lame (14') avec un tranchant (14") acéré, la lame de coupe (14) étant disposée à l'intérieur d'une fenêtre de coupe (15) ouverte de manière continue dans une direction (x') transversale à la direction de l'axe longitudinal (z), la lame de coupe (14) étant positionnée dans la fenêtre de coupe (15) de telle sorte que son tranchant (14") acéré soit parallèle à un axe transversal (y) qui s'étend perpendiculairement à l'axe longitudinal (z) et obliquement par rapport à la direction (x'), le tranchant (14") acéré de la lame de coupe (14) est positionné axialement à partir du corps de lame (14') dans la direction de l'axe longitudinal (z) vers la poignée en forme de manche (12), la lame de coupe (14) étant reliée au corps de dispositif (11) de manière rigide ou mobile autour de l'axe transversal (y), et la fenêtre de coupe (15) et la lame de coupe (14) sont logées dans un corps de coupe (13') relié de manière rigide au corps du dispositif (11),
**caractérisé en ce**
**que** le corps de coupe (13') comporte une entretoise (16) qui définit la profondeur de pénétration du bord tranchant (14") acéré dans le tissu propre (19) du patient et donc l'épaisseur de la fine lamelle de tissu ou de cartilage (19') prélevée;
et **que** l'entretoise (16) est dessinée réglable.

2. Dispositif de coupe selon la revendication 1, **caractérisé en ce que** la fenêtre de coupe (15) et la lame de coupe (14) sont logées dans un corps de coupe (13') relié d'un seul tenant au corps du dispositif (11).

3. Dispositif de coupe selon la revendication 2, **caractérisé en ce que** l'entretoise (16) comprend une ou plusieurs rondelles d'écartement (16') qui ont notamment une épaisseur comprise entre 0,1 mm et 1 mm, de préférence entre 0,3 mm et 0,4 mm.

4. Dispositif de coupe selon l'une quelconque des revendications 2 ou 3, **caractérisé en ce que** la tête de coupe (13) est décalée axialement par rapport à l'axe longitudinal (z) en direction de la poignée en forme de manche (12), à la suite du corps de coupe (13') dans la direction de l'axe longitudinal (z) vers la poignée en forme de manche (12), présente une fenêtre d'observation (17) continue dans la direction d'un axe vertical (x) perpendiculaire à l'axe longitudinal (z) et perpendiculaire à l'axe transversal (y).

5. Dispositif de coupe selon l'une quelconque des revendications précédentes, **caractérisé en ce qu'**un dispositif de perforation du cartilage (18) est disposé à l'extrémité libre de la poignée en forme de manche (12) opposée à la tête de coupe (13).

6. Dispositif de coupe selon l'une quelconque des revendications précédentes, **caractérisé en ce que** la lame de coupe (14) est disposée dans le corps de coupe (13') avec le côté de son tranchant (14") acéré incliné par rapport au plan z-y selon un angle d'attaque compris entre 10° et 40°, de préférence d'environ 25°.

7. Dispositif de coupe selon l'une quelconque des revendications précédentes, **caractérisé en ce que** la lame de coupe (14) est fabriquée à partir d'une lame de rasoir.

8. Dispositif de coupe selon l'une quelconque des revendications précédentes, **caractérisé en ce que** la lame de coupe (14) est fabriquée en acier inoxydable, de préférence en acier chirurgical.

9. Dispositif de coupe selon la revendication 7 ou 8, **caractérisé en ce que** le tranchant (14") acéré de la lame de coupe (14) présente un affûtage des deux côtés et est de préférence trempé.

10. Dispositif de coupe selon l'une quelconque des revendications 1 à 9, **caractérisé en ce que** l'ensemble du corps (11) du dispositif de coupe (10; 20) est fabriqué en acier inoxydable, de préférence en acier chirurgical.

11. Dispositif de coupe selon l'une quelconque des revendications 1 à 9, **caractérisé en ce que** des parties du dispositif de coupe (10; 20), en particulier la poignée en forme de manche (12), sont fabriquées en matière plastique.

12. Dispositif de coupe selon l'une quelconque des revendications précédentes, **caractérisé en ce que** la poignée en forme de manche (12) présente une structure creuse ou concave.

13. Dispositif de coupe selon l'une quelconque des revendications précédentes, **caractérisé en ce que** le tranchant (14") acéré de la lame de coupe (14) présente une forme en poutre ou en dent de scie ou en faucille ou ovale ou ronde, concave ou convexe, en particulier sur une section circulaire, de préférence sur un quart ou un demi-cercle.
